# EUROPEAN PATENT APPLICATION

(11) **EP 2 626 423 A1**
(43) Date of publication of application: **14.08.2013**
(21) Application number: 12000783.6
(22) Date of filing: 07.02.2012
(51) Int. Cl.: C12N 9/90, C12P 7/06, C12P 19/24

(54) **Pentose-fermenting microorganism**

(71) Applicant: Clariant Produkte (Deutschland) GmbH, 65929 Frankfurt/Main (DE)
(72) Inventor: Dragovic, Zdravko, Dr., 81375 München (DE); Gamauf, Christian, Dr., 81477 München (DE); Reisinger, Christoph, Dr., 81477 München (DE)
(74) Representative: HOFFMANN EITLE

(57) **Abstract**

The present invention relates to a microbial eukaryotic cell capable of utilizing C5 sugars, in particular xylose. The present invention further relates to a protein sequence to enable eukaryotic cells to degrade C5 sugars.

## Description

### Field of invention

Xylose is a major building block of plant biomass, and finds itself bound in a number of major feedstock in focus by nowadays biorefinery concepts. Examples for such xylose-rich materials include wheat straw, corn stover or wood chips or other wood by-products (Blake A. et al. Genome Biol. 2008; 9(12): 242).

As a consequence a performed hydrolysis of the starting material by enzymatic, chemical or chemo/enzymatic approaches leads to intermediate products rich in xylose, besides other valuable sugars (Deepak Kumar, et al. Biotechnol Biofuels. 2011; 4: 27). The efficient utilization of C5 rich sugar solutions in coupled fermentation lines is both crucial and demanding for the applied fermentation strains (Sara Fernandes, Patrick Murray Bioeng Bugs. 2010 Nov-Dec; 1(6): 424-428). Especially C6 yeasts, such as *Saccharomyces cerevisiae,* that are desired working horses due to the long history of breeding that ended up in traits with extreme ethanol tolerance and high yields for glucose conversion, leave xylose completely untouched, thereby decreasing the potential yield. Several strategies are known to circumvent this limitation. A key step herein appears the successful feeding of the xylose by isomerisation into xylulose and subsequent modification cascade of the non reductive part of the C5 shunt into the regular glycolysis pathway of Saccharomyces cerevisiae. While strength of xylose uptake through membrane by specific transporters and the achievable flux density through the C5 shunt are subject to possible enhancements (David Runquist, et al. Microb Cell Fact. 2009; 8: 49. ), the key isomerization step from xylose to xylulose poses a major problem in the overall process. Two principle pathways are known to perform the step. The first, employing subsequent steps of reduction to xylitol (by xylose reductase) and oxidation (by xylitol dehydrogenase) to xylulose, causes a major imbalance between NADH and NADPH cofactors and leads to increased formation of xylitol under fermentation conditions (Maurizio Bettiga, et al. Biotechnol Biofuels. 2008; 1: 16.). The alternative direct isomerization by application of xylose isomerase suffers from the lack of availability of xylose isomerase genes combining an active expression in eukaryotic microorganisms (in particular yeasts like Saccharomyces cerevisiae), a high catalytic efficiency, a temperature optimum adapted to the fermentation temperature and a low inhibition by side products, especially xylitol. One aspect of the present invention is the disclosure of protein sequences and their nucleic acids encoding the same, to fulfill this requirement.

The xylose isomerase pathway is native to bacterial species and to rare yeasts. In contrast to oxidoreductase pathway the isomerase pathway requires no cofactors. The isomerase pathway minimally consists of single enzymes, heterologous xylose isomerases (XI), which directly convert xylose to xylulose. As with the oxidoreductase pathway, the further improvement of the yield can be obtained by coexpression of heterologous xylulose kinase (XK).

First functionally expressed XI was a xylA gene from anaerobic fungus Piromyces sp E2 (Kuyper M, et al. FEMS Yeast Res. 2003 Oct;4(1):69-78.). The haploid yeast strain with ability to ferment xylose as a sole carbon source under anaerobic conditions was constructed. The majority of xylose isomerases are bacterial proteins and a major obstacle was their expression in yeast. However recent work has demonstrated functional expression in yeast (Table 1).

**Table 1: Examples for Xylose isomerases claimed for the application in yeasts**

| **Source Organism** | **Citation** |
|---|---|
| *Clostridium phytofermentas* | WO 2010/000464 |
| *Piromyces sp. E2* | EP 1 468 093 B1 |

Sugar transport across the membrane does not limit the fermentation of hexose sugars, although it may limit pentose metabolism especially in case of hexose and pentose cofermentations. Several pentose transporter expression studies have been performed.

### Brief description of the invention

An objective of the invention is to provide a microbial eukaryotic cell capable of utilizing C5 sugars, in particular xylose. Another objective of the invention is to provide a protein sequence to enable eukaryotic cells to degrade C5 sugars.

It was surprisingly found that the protein described by Seq.ID NO. 2 (sequence previously published in NCBI GeneBank accession number ZP_07904696.1) is functionally expressed in eukaryotic microbial cells, in particular yeasts like Saccharomyces cerevisiae, when these cells are transformed with a vector carrying an expression cassette comprising a DNA sequence coding for said Seq. ID No. 2 Protein, for example the DNA Molecule described under Seq. ID.NO 1 (previously published in Gene Bank as part of Accession Number NZ_AEPW01000073.1 GI:315651683).

It was further found, that transformed cells show an increased rate of xylose consumption when compared to the non-transformed cells.

As a further aspect, the fermentation of biomass from xylose carbon source containing media was improved and the amount of metabolites formed by such transformed strains under these conditions was increased compared to transformed controls.

Another aspect of the invention relates to the biocatalytic properties of the expressed protein and its application as biocatalyst in situ or in purified form for the production of isomerized sugar products or intermediates.

### Description

### Definition

Xylose isomerase activity is herein defined as the enzymatic activity of an enzyme belonging to the class of Xylose isomerases (EC 5.3.1.5), thus catalyzing the isomerisation of various aldose and ketose sugars and other enzymatic side reactions inherent to this class of enzymes. The assignment of a protein to the class of xylose isomerase is either performed based on activity pattern or homology considerations, whatever is more relevant in each case. Xylose isomerase activity can be determined by the use of a coupled enzymatic photometric assay employing sorbitol dehydrogenase.

An expression construct herein is defined as a DNA sequence comprising all required sequence elements for establishing expression of an comprised open reading frame (ORF) in the host cell including sequences for transcription initiation (promoters), termination and regulation, sites for translation initiation, regions for stable replication or integration into the host genome and a selectable genetic marker. The functional setup thereby can be already established or reached by arranging (integration etc.) event in the host cell. In a preferred embodiment the expression construct contains a promoter functionally linked to the open reading frame followed by an optional termination sequence. Preferred promoters are medium to high strength promoters of Saccharomyces cerevisiae, active under fermentative conditions. Examples for such preferred promoters are promoters of the glycolytic pathway or the sugar transport, particularly the promoters of the genes known as PFK1, FBA1, PGK1, ADH1, ADH2, TDH3 as well truncated or mutated variants thereof. Elements for the establishment of mitotic stability are known to the art and comprise S. cerevisiae 2µ plasmid origin of replication, centromeric sequences (CEN), autonomous replicating sequence (ARS) or homologous sequences of any length for the promotion of chromosomal integration via the homologous end joining pathway. Selectable markers include genetic elements referring antibiotic resistance to the host cell. Examples are Kan, *ble.* Marker genes. Auxotrophy markers complementing defined auxotrophies of the host strain can be used. Examples for such markers to be mentioned are genes and mutations reflecting the leucine (LEU2) or uracil (URA3) pathway, but also xylose isomerase.

Enhanced xylose consumption is herein defined as any xylose consumption rate resulting in cell growth and proliferation, metabolite formation and or caloric energy generation which is increased in comparison to the xylose consumption rate of the non-modified cell (culture) with respect to the considered trait. The consumption rate can be determined for instance phenomenologically by consideration of formed cell density or colony size, by determination of oxygen consumption rate, formation rate of ethanol or by direct measurement of xylose concentration in the growth media over time. Consumption in this context is equivalent to the terms utilization, fermentation or degradation.

Genes involved in the xylose metabolism were described by various authors and encode hexose and pentose transporters, xylulokinase, ribulose-5-phosphate-3-epimerase, ribulose-5-phosphate isomerase, transketolase, transaldolase and homologous genes.

Xylose isomerase expressing cell herein is referred to as a microbial eukaryotic cell which was genetically modified in carrying an expression construct for the expression of the disclosed xylose isomerase. In a preferred embodiment the xylose isomerase expressing cell is a yeast selected from the group of Pichia, Pachysolen, Yarrowia, Saccharomyces, Candida, Arxula, Ashbya, Debaryomyces, Hansenula, Hartaea, Kluyveromyces, Schwanniomyces, Trichosporon, Xanthophylomyces, Schizosaccharomyces, Zygosaccharomyces, most preferably being Saccharomyces cerevisiae.

### Detailed description of the of invention

The present invention provides solutions for the genetic construction of eukaryotic cells with an enhanced xylose metabolism, an improved biomass formation in the presence of xylose and/or improved formation of metabolites. These are desirable properties and present bottlenecks for many industrial production strains, especially production strains of the genus Saccharomyces, to name Saccharomyces cerevisiae as non limiting example.

The invention solves this problem by providing Protein and DNA sequences of xylose isomerase genes that are functionally expressed in lower eukaryotic cells, especially yeasts with an outlined example being yeasts of the genus Saccharomyces, again to name Saccharomyces cerevisiae as non limiting example. The created strain is an xylose isomerase expressing cell showing a potentially enhanced xylose consumption. The desired property of xylose isomerase activity produced by the xylose isomerase expressing cell is difficult to realize in a satisfactory manner with means known to the art. The identity of the functional xylose isomerase protein is therefore disclosed as homologous proteins to Seq. ID NO 2 showing at least 70%, 80% 90% or 95% identity to Seq.ID. NO 2 or being identical to the Seq.ID. NO 2, as is part of the given invention. Identity levels can be determined by the computer program AlignX sold in the Vector-NTI-Package by Life^{tm} Technology. The functional xylose isomerase protein is also disclosed as proteins having mutations to SEQ ID NO: 2 while having xylose isomerase activity or as proteins encoded by DNA sequences having mutations to SEQ ID NO: 1. The mutations may be based on, for example, substitutions, deletions, or insertions of one or more amino acid residues at one or more positions in SEQ ID. NO 2 or one or more nucleotides to one or more positions in SEQ ID. NO 1. The number of mutations is preferably 1-50, more preferably 1-30, most preferred 1-20, and most highly preferred 1-10 for amino acid residues and preferably 1-150, more preferably 1-100, most preferred 1-50, most highly preferred 1-10 for nucleotides.

It is clear to the skilled person that high numbers of varying DNA molecules translate to the same protein sequence and shall be covered by the invention as such. A preferred example of DNA sequence is given in Seq. ID NO 1. Methods for computational enhancement of a DNA-sequence with respect to protein production levels are known. They nonexclusively include methods employing statistic evaluation of preferred codons (Codon usage tables), mRNA secondary-structure predicting algorithms and knowledge based models based on HMM or NN. In such way optimized DNA sequences calculated from the targeted protein sequence are preferred and included in the invention. Also included in the invention are DNA sequences obtained by recursive or non recursive steps of mutagenesis and selection or screening of improved variants. This is a regular technique for the improvement of DNA and protein sequences and sequences obtained by such methods cannot be excluded from the inventive concept. This shall be seen independent from the question whether the outcoming DNA sequence of such an experiment leaves the translated protein sequence untouched or translates to mutations in them, as long as the levels of identity do not fall below preferably 70%, 80% 90% or 95% to Seq ID NO 2. A preferred embodiment of the invention indeed applies such processes of improvements for the adjustment of the disclosed nucleic acid molecules and protein sequences to the particular problem.

Another aspect of the invention relates to chimeric sequences generated by fusions of parts of the inventive xylose isomerase sequence with parts of other proteins, preferably parts of such proteins showing high identity levels to known xylose isomerases or demonstrated xylose isomerase activity themselves as well as nucleic acid molecules encoding such chimeric proteins. Especially fusions of the N-terminal part of Seq ID NO 2 Protein or the 5'-part of the Seq. ID NO 1 Nucleic acid Molecule shall be highlighted as preferred embodiments of the present invention.

It has been in the field of vision of the inventors that the step of the xylose isomeration as solved by the invention is one central change required and for the setup of an efficient carbon flux with xylose as starting block further changes the xylose isomerase expressing cell might be necessary. Issues known to the authors include xylose trans-membrane transport, especially uptake from the growth medium, the phoshorylation and the metabolic steps of the C5 shunt (non-oxidative part of the pentose phosphate shunt). Therefore additional changes introduced into the cell, especially those reflecting emendation of the known issues and alter expression levels of genes involved in the xylose metabolism, present a preferred embodiment of the invention. The order of introductions of such changes to the cells, which can be done subsequent or parallel in random or ordered manners, shall not be distinguished at this point and all possible strategies are seen as integral part of and as special embodiments of the invention.

A further aspect of the invention relates to the application of the xylose isomerase expressing cell for_the production of biochemicals based on xylose containing raw material. Biochemicals include biofuels like ethanol or butanol as well as bio-based raw materials for bulk chemicals like lactic acid, itaconic acid to name some examples. A list of possible biochemicals was published by US department of energy.

### Examples

### Identification of candidate gene sequences with xylose isomerase function

For the finding of xylose isomerase sequences within Genebank the program BlastP (Stephen F. Altschul, et al. (1997), Nucleic Acids Res. 25:3389-3402.) at the NCBI genomic BLAST site (http://www.ncbi.nlm.nih.gov/sutils/genom_table.cgi) were chosen. As a test sequence the protein sequence of the *Escherichia coli* K12 xylose isomerase gene (Seq ID. NO 3) was taken as query sequence. Standard parameters of the program were not modified and the query was blasted against the bacterial protein databases including Eubacterium saburreum DSM 3986 database built on the results of the shotgun sequence of the organism (accession numer) NZ_AEPW01000000. Sequences with significant homology level over the whole sequence length were taken into account. The search revealed a number of potential candidate genes which were subsequently cloned into S. cerevisiae and tested for functional expression. All such candidate genes were treated as Entry ZP_07904696.1 (Seq ID. No 2) which is a xylose isomerase gene (EsXI) from Eubacterium saburreum DSM 3986 as described in the following paragraphs. The linked coding sequence entry (NZ_AEPW01000073 REGION: 2583..3956; Seq ID NO 1) was taken as basis for the construction of cloning primers.

### Amplification of Eubacterium saburreum DSM 3986 xylose isomerase gene (EsXI)

Methods for manipulation of nucleic acid molecules are generally known to the skilled person in the field and are here introduced by reference (1. Molecular cloning: a laboratory manual, Joseph Sambrook, David William Russell*; 2.* Current Protocols in Molecular Biology. Last Update: January 11, 2012. Page Count: approx. 5300. Print ISSN: 1934-3639*;.).* Genomic template DNA of ***Eubacterium saburreum DSM 3986*** was purchased from DSMZ (Deutsche Stammsammlung für Mikroorganismen und Zellkulturen). Flanking Primer pairs were designed to match the N- and C-terminal ending of Seq ID NO1. The PCR reaction is set up using Finnzymes Phusion^{tm} High Fidelity Polymerase (HF-Buffer system) following the recommendations of the supplier for dNTP, primer and buffer concentrations. The amplification of the PCR-product is done in an Eppendorf Thermocycler using the standard program for Phusion Polymeras (98°C 30" initial denaturation followed by 35 cycles of 98°C (20") - 60°C (20") - 72°C (1'20") steps and a final elongation phase at 72°C for 10 minutes. The PCR product of expected size is purified by preparative ethidium bromide stained TAE-Agarose gel electrophoresis and recovered from the Gel using the Promga Wizard SV-PCR and Gel Purification Kit. For the fusion of a C-terminal 6xHis-Tag the primary PCR-product is used as template for re-amplification of the whole DNA fragment using an extended reverse Primer with corresponding 5' extension, under identical conditions (6xHIS-Tag fusion PCR). The PCR product obtained is again purified by Agarose Gel Electrophoresis and recovered using the Promga Wizard SV-PCR and Gel Purification Kit. It contains the C-terminal 6x-His TAG Version of the *Es*XI-gene.

### Cloning of the EsXI ORF into Saccharomyces cerevisiae expression plasmid

A plasmid preparation of the pSCMB454 Plasmid isolated from an Escherichia coli culture was linearized by restriction with *Xmn*I Endonuclease and digested fragments separated from unprocessed species by Agarose Gel Electrophoresis. The linearized Vector-Backbone was recovered from the gel following the instructions of the Promga Wizard SV-PCR and Gel Purification Kit. The amplified PCR Product is cloned into the *Xmn*I digested Vector-backbone using standard cloning methods. Transformation was performed into chemically competent *Escherichia coli* W Mach1 cells according to the suppliers Protocol. Transformants were grown over night on LB-Ampicillin Plates and tested for correctness by plasmid MINI-prep and control digestion as well as DNA sequencing. A larger quantity of Plasmid DNA was prepared from a confirmed clone using the Promega PureYield™ Plasmid Midiprep System. The sequence of the resulting expression cassette including *GPD*-promoter-sequnece and cyc1 terminator is given in Seq. ID NO 6.

### Transformation in Saccharomyces cerevisiae

*Saccharomyces cerevisiae* strain ATCC 204667 (MATa, ura3-52, mal GAL+, CUP(r)) was used as host for all transformation experiments.

Transformations is performed using standard methods known to those skilled in the art (e.g. see Gietz, R.D. and R.A. Woods. (2002) Transformation of yeast by the LiAc/ss carrier DNA/ PEG method. Methods in Enzymology 350: 87-96) . An intact version of the *S. cerevisiae ura3* gene contained in the expression vector was used as selection marker and transformants are selected for growth on minimal medium without uracil. Minimal medium consisted of 20 g·l⁻¹ glucose, 6.7 g·l⁻¹ yeast nitrogen base without amino acids, 40 mg·l⁻¹ L-tyrosine, 70 mg·l⁻¹ L-phenylalanine, 70 mg·l⁻¹ L-tryptophane, 200 mg·l⁻¹ L-valine and 50 mg·l⁻¹ each of adenin hemisulfate, L-arginine hydrochloride, L-histidine hydrochloride monohydrate, L-isoleucine, L-leucine, L-lysine hydrochloride, L-methionine, L-serine and L-threonine. The pH was adjusted to 5.6 and 15 g·l⁻¹ agar is added for solid media.

### Growth of Saccharomyces cerevisiae cells expressing EsXI on xylose medium

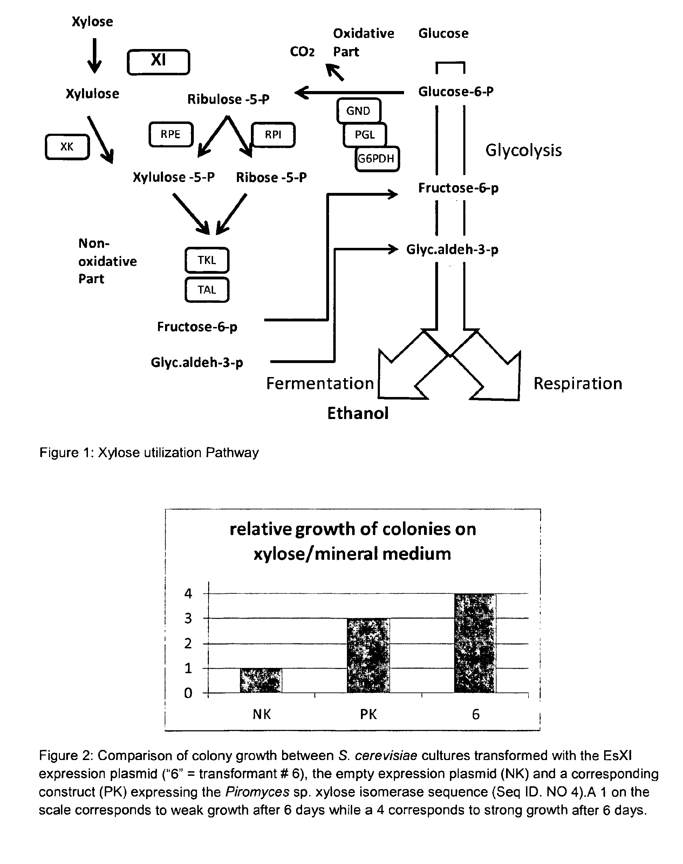

Single colonies of transformants with the EsXI expression vector as well as with the empty expression vector were transferred on minimal medium plates to test the stability. Single colonies were then transferred on minimal medium plates with 20 g·l⁻¹ xylose instead of glucose and incubated at 30 °C. After 6- 7 days only the transformants with the EsXI expression vector were visibly growing (see Fig. 2).

### Sequence listing

Seq. ID NO 1: Sequence of Eubacterium sabbureum DSM 3986 DNA sequence encoding xylose isomerase (NZ_AEPW01000073.1 GI:315651683) 
gtgaaaacaaaaaacaacattatatgtactattgcattgaaaggagacatatttatgaaagaattttttcccggcatatcacctgtaaa
gtttgagggcagagatagtaaaaatccacttagtttcaaatattatgatgccaaaagggtgataatgggcaaaacaatggaggaac
atttatcatttgctatggcatggtggcataatctttgtgcctgtggtgtggatatgttcggacagggtactgtcgataaaagttttggtg
aaagctccggtactatggagcatgcaagggctaaagtggatgcaggcattgaatttatgaaaaagcttggtataaagtattattgct
tccatgatacggatattgtacctgaggatcaggaagatataaatgttaccaatgcacgtttggatgagattacagactatatcttaga
aaaaacaaaggataccgatattaaatgtctttggacaacctgcaatatgttcagtaatccaagatttatgaacggtgcaggaagctc
aaacagtgcagatgtattttgctttgcagcggcacaggcaaagaaaggtcttgaaaatgccgtaaaacttggagcaaagggattt
gtattctggggaggcagagaaggttatgagacacttctaaatacagatatgaagcttgaagaggaaaatatagcaacactctttac
aatgtgcagagattatggacgcagtataggctttatgggagattttatattgagcctaagccgaaggagcctatgaagcatcagta
tgattttgatgcggcaactgcaatcggtttttaagaaaatatggacttgataaagatttcaaactaaatattgaggcaaatcacgcta
cacttgcaggtcatacttttcagcatgagttaagagtatgtgcagtcaacggtatgatggggtcggtagatgccaatcaaggagat
acattacttggatgggacactgatcaattccctacaaatgtctatgatactacattggctatgtatgaaatattaaaggcaggcggac
tccgtggaggtctgaactttgattcaaagaatcgcagaccaagtaatacagccgatgatatgttctatggctttatagcaggtatgg
acacatttgcacttggacttattaaggcggcggaaattatagaagacggaagaatagatgattttgttaaagaaagatatgcaagtt
ataattcaggaataggtaagaagataagaaacagaaaagtgacactgatagagtgtgccgagtatgccgcaaagcttaaaaagc
ctgaactgccggaatcaggaagacaggaatatcttgagagcgtagtgaataatatattgttcggataa
Seq. ID NO 2: Protein Sequence of translated Eubacterium sabbureum DSM 3986 DNA sequence encoding xylose isomerase (ZP_07904696.1) 
mktknniictialkgdifmkeffpgispvkfegrdsknplsfkyydakrvimgktmeehlsfamawwhnlcacgvdmfg
qgtvdksfgessgtmeharakvdagiefmkklgikyycfhdtdivpedqedinvtnarldeitdyilektkdtdikclwttcn
mfsnprfmngagssnsadvfcfaaaqakkglenavklgakgfvfwggregyetllntdmkleeeniatlftmcrdygrsig
fmgdfyiepkpkepmkhqydfdaataigflrkygldkdfklnieanhatlaghtfqhelrvcavngmmgsvdanqgdtll
gwdtdqfptnvydttlamyeilkagglrgglnfdsknrrpsntaddmfygfiagmdtfalglikaaeiiedgriddfvkerya
synsgigkkirnrkvtliecaeyaaklkkpelpesgrqeylesvvnnilfg*
Seq. ID NO 3: *Eschericha coli* xylose isomerase (Protein) 
mqayfdqldrvryegskssnplafrhynpdelvlgkrmeehlrfaacywhtfcwngadmfgvgafnrpwqqpgealala
krkadvafeffhklhvpfycfhdvdvspegaslkeyinnfaqmvdvlagkqeesgvkllwgtancftnprygagaatnpd
pevfswaatqvvtameathklggenyvlwggregyetllntdlrqereqlgrfmqmvvehkhkigfqgtlliepkpqeptk
hqydydaatvygflkqfglekeiklnieanhatlaghsfhheiataialglfgsvdanrgdaqlgwdtdqfpnsveenalvm
yeilkaggfttgglnfdakvrrqstdkydlfyghigamdtmalalkiaarmiedgeldkriaqrysgwnselgqqilkgqms
ladlakyaqehhlspvhqsgrqeqlenlvnhylfdk*
Seq. ID NO 4: *Clostridium phytofermentas* xylose isomerase (Protein) 
mknyfpnvpevkyegpnstnpfafkyydankvvagktmkehcrfalswwhtlcaggadpfgvttmdrtygnitdpmel
akakvdagfelmtklgieffcfhdadiapegdtfeeskknlfeivdyikekmdqtgikllwgtannfshprfmhgastscna
dvfayaaakiknaldatiklggkgyvfwggregyetllntdlgleldnmarlmkmaveygrangfdgdfyiepkpkeptk
hqydfdtatvlaflrkyglekdfkmnieanhatlaghtfehelamarvngafgsvdanqgdpnlgwdtdqfptdvhsatla
mlevlkaggftngglnfdakvrrgsfefddiaygyiagmdtfalglikaaeiiddgriakfvddryasyktgigkaivdgttsle
eleqyvlthsepvmqsgrqevletivnnilfr*
Seq. ID NO 5: *Piromyce sp.* xylose isomerase (Protein) 
makeyfpqiqkikfegkdsknplafhyydaekevmgkkmkdwlrfamawwhtlcaegadqfgggtksfpwnegtda
ieiakqkvdagfeimqklgipyycfhdvdlvsegnsieeyesnlkavvaylkekqketgikllwstanvfghkrymngast
npdfdvvaraivqiknaidagielgaenyvfwggregymsllntdqkrekehmatmltmardyarskgfkgtfliepkpm
eptkhqydvdtetaigflkahnldkdfkvnievnhatlaghtfehelacavdagmlgsidanrgdyqngwdtdqfpidqye
lvqawmeiirgggfvtggtnfdaktrrnstdlediiiahvsgmdamaralenaakllqespytkmkkeryasfdsgigkdfe
dgkltleqvyeygkkngepkqtsgkqelyeaivamyq*
Seq. ID NO 6: *Es*XI Expression cassette (**BOLD CAPITALS**: coding sequence of the *Es*XI Gene with C-terminal 6x-His-Tag and linker fusion; SMALL CAPITALS: *GPD-*promoter; underlinded: remains of *Xnm*I site; *italic:* CYC1 terminator)

CTCGCCATTTCAAAGAATACGTAAATAATTAATAGTAGTGATTTTCCTAACTTTATTTAGTCAAAA
AATTAGCCTTTTAATTCTGCTGTAACCCGTACATGCCCAAAATAGGGGGCGGGTTACACAGAATAT
ATAACATCGTAGGTGTCTGGGTGAACAGTTTATTCCTGGCATCCACTAAATATAATGGAGCCCGCT
TTTTAAGCTGGCATCCAGAAAAAAAAAGAATCCCAGCACCAAAATATTGTTTTCTTCACCAACCAT
CAGTTCATAGGTCCATTCTCTTAGCGCAACTACAGAGAACAGGGGCACAAACAGGCAAAAAACGG
GCACAACCTCAATGGAGTGATGCAACCTGCCTGGAGTAAATGATGACACAAGGCAATTGACCCAC

GCATGTATCTATCTCATTTTCTTACACCTTCTATTACCTTCTGCTCTCTCTGATTTGGAAAAAGCTGA
AAAAAAAGGTTGAAAGCAGTTCCCTCAAATTATTCCCCTACTTGACTAATAAGTATATAAAGACGG
TAGGTATTGATTGTAATTCTGTAAATCTATTTCTTAAACTTCTTAAATTCTACTTTTATAGTTAGTCT
TTTTTTTAGTTTTAAAACACCAAGAACTTAGTTTCGAATaaacacacagaaacaaagaaaATGAAAAC
AAAAAACAACATTATATGTACTATTGCATTGAAAGGAGACATATTTATGA
AAGAATTTTTCCCGGCATATCACCTGTAAAGTTTGAGGCAGAGATAGT
AAAAATCCACTTAGTTTCAAATATTATGATGCCAAAAGGGTGATAATGGG
CAAAACAATGGAGGAACATTTATCATTTGCTATGGCATGGTGGCATAATC
TTTGTGCCTGTGGTGTGGATATGTTCGGACAGGGTACTGTCGATAAAAG
TTTTGGTGAAAGCTCCGGTACTATGGAGCATGCAAGGGCTAAAGTGGAT
GCAGGCATTGAATTTATGAAAAAGCTTGGTATAAAGTATTATTGCTTCCA
TGATACGGATATTGTACCTGAGGATCAGGAAGATATAAATGTTACCAATG
CACGTTTGGATGAGATTACAGACTATATCTTAGAAAAACAAAGGATACC
GATATTAAATGTCTTTGGACAACCTGCAATATGTTCAGTAATCCAAGATT
TATGAACGGTGCAGGAAGCTCAAACAGTGCAGATGTATTTTGCTTTGCAG
CGGCACAGGCAAAGAAAGGTCTTGAAAATGCCGTAAAACTTGGAGCAAA
GGGATTTGTATTCTGGGGAGGCAGAGAAGGTTATGAGACACTTCTAAAT
ACAGATATGAAGCTTGAAGAGGAAAATATAGCAACACTCTTTACAATGTG
CAGAGATTATGGACGCAGTATAGGCTTTATGGAGATTTTATATTGAGC
CTAAGCCGAAGGAGCCTATGAAGCATCAGTATGATTTTGATGCGGCAAC
TGCAATCGGTTTTTTAAGAAAATATGGACTTGATAAAGATTTCAAACTAA
ATATTGAGGCAAATCACGCTACACTTGCAGGTCATACTTTTCAGCATGAG
TTAAGAGTATGTGCAGTCAACGGTATGATGGGGTCGGTAGATGCCAATC
AAGGAGATACATTACTTGGATGGGACACTGATCAATTCCCTACAAATGTC
TATGATACTACATTGGCTATGTATGAAATATTAAAGGCAGGCGGACTCCG
TGGAGGTCTGAACTTTGATTCAAAGAATCGCAGACCAAGTAATACAGCC
GATGATATGTTCTATGGCTTTATAGCAGGTATGGACACATTTGCACTTGG
ACTTATTAAGGCGGCGGAAATTATAGAAGACGGAAGAATAGATGATTTT
GTTAAAGAAAGATATGCAAGTTATAATTCAGGAATAGGTAAGAAGATAAG
AAACAGAAAAGTGACACTGATAGAGTGTGCCGAGTATGCCGCAAAGCTT
AAAAAGCCTGAACTGCCGGAATCAGGAAGACAGGAATATCTTGAGAGCG
TAGTGAATAATATATTGTTCGGAGGATCTGGCCATCACCACCATCATCAC
TAAtgttcgtcctcgtttagttatgtcacgcttacattcacgccctcccccacatccgctctaaccgaaaggaaggagtta
gacaacctgaagtctaggtccctatttattttttatagttatgttagtattaagaacgttatttatatttcaaattttcttttttctgta
cagacgcgtgtacgcatgtaacattatactgaaaaccttgcttgagaaggttttgggacgctcgaaggctttaatttg

### Figures

Figure 1: Xylose utilization Pathway
Figure 2: Comparison of colony growth between *S*. *cerevisiae* cultures transformed with the EsXI expression plasmid ("6" = transformant # 6), the empty expression plasmid (negative control = NK) and a corresponding construct (PK) expressing the *Piromyces* sp. xylose isomerase sequence (Seq ID. NO 4)

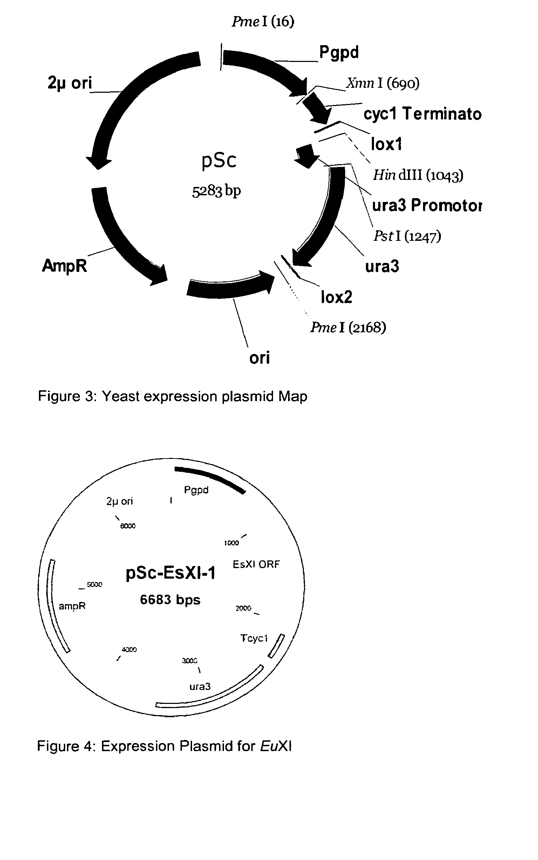

Figure 3: Yeast expression plasmid Map
Figure 4: Expression Plasmid for *Eu*XI

## Claims

1. A protein having at least 70% identity, preferably 80% identity, most preferably 90 % identity, most highly preferably 95 % identity to SEQ ID NO. 2 and having xylose-isomerase activity in a eukaryotic cell.

2. The protein of claim 1, wherein the protein consists of the sequence of SEQ ID NO. 2.

3. A DNA molecule encoding a protein of at least 70% identity, preferably 80% identity, most preferably 90 % identity, most highly preferably 95 % identity to SEQ ID NO. 2, wherein said protein has xylose-isomerase activity in said eukaryotic cell.

4. The DNA molecule of claim 3, wherein the DNA molecule consists of the sequence of SEQ ID NO. 1.

5. A eukaryotic cell comprising a DNA molecule from claims 3 or 4.

6. A eukaryotic cell expressing a protein from claims 1 or 2.

7. The eukaryotic cell of claim 6, wherein the eukaryotic cell is a yeast cell, preferably selected from the group of Pichia, Pachysolen, Yarrowia, Saccharomyces, Candida, Arxula, Ashbya, Debaryomyces, Hansenula, Hartaea, Kluyveromyces, Schwanniomyces, Trichosporon, Xanthophylomyces, Schizosaccharomyces, Zygosaccharomyces, most preferably being Saccharomyces cerevisiae.

8. A genetically modified yeast cell comprising an exogenous xylose isomerase gene functional in said yeast cell, preferably wherein the exogenous xylose isomerase gene is operatively linked to promoter and terminator sequences that are functional in said yeast cell.

9. The genetically modified yeast cell of claim 8 wherein the exogenous xylose isomerase gene is a DNA molecule from claims 3 or 4.

10. The genetically modified yeast cell of claims 8 or 9, wherein the genetically modified yeast cell is selected from the group of Pichia, Pachysolen, Yarrowia, Saccharomyces, Candida, Arxula, Ashbya, Debaryomyces, Hansenula, Hartaea, Kluyveromyces, Schwanniomyces, Trichosporon, Xanthophylomyces, Schizosaccharomyces, Zygosaccharomyces, preferably being Saccharomyces cerevisiae.

11. A eukaryotic cell having increased levels of xylose isomerase activity obtained by transformation of a wild type yeast strain with a DNA comprising a DNA sequence encoding a protein of at least 70% identity, preferably 80% identity, most preferably 90 % identity, most highly preferably 95 % identity to SEQ ID NO. 2.

12. The eukaryotic cell of claim 11, wherein the protein consists of the sequence of SEQ ID NO. 2.

13. The eukaryotic cell of claim 11 or 12, wherein the yeast strain is selected from the group of Pichia, Pachysolen, Yarrowia, Saccharomyces, Candida, Arxula, Ashbya, Debaryomyces, Hansenula, Hartaea, Kluyveromyces, Schwanniomyces, Trichosporon, Xanthophylomyces, Schizosaccharomyces, Zygosaccharomyces, preferably being Saccharomyces cerevisiae.

14. Use of the protein from claim 1 or 2 or the cells from any one of claims 5-13 for fermentation of biomass from a xylose-carbon source containing media.

15. Use of the protein from claim 1 or 2 or the cells from any one of claims 5-13 as a biocatalyst in situ or in purified form for the production of isomerized sugar products or intermediates, preferably for isomerized sugar products.

16. Use of a DNA molecule from claims 3 or 4 for transformation of a eukaryotic cell.

17. Use of claim 16, wherein the transformation results in a eukaryotic cell of any one of claims 5, 6, 7, or 11-13 or genetically modified yeast cells from any one of claims 8-10, preferably eukaryotic cells from any one of claims 5, 6, 7, or 11-13.

18. Use of the eukaryotic cells from any one of claims 5, 6, 7, or 11-13 or genetically modified yeast cells from any one of claims 8-10, most preferably the eukaryotic cells from any one of claims 5, 6, 7, or 11-13 for achieving an increased rate of xylose consumption.

19. Process for producing ethanol from xylose or a glucose-xylose mixture using a yeast, preferably Saccharomyces cerevisiae, which expresses the protein of claim 1 or 2.

20. Process for producing a fermentation product selected from the group of lactic acid, acetic acid, succinic acid, amino acids, 1, 3-propane-diol, ethylene, glycerol, ß-lactam antibiotics, cephalosporins, biofuels, butanol, ethanol, lactic acid, itaconic acid, preferably butanol, most preferably ethanol whereby the process comprises the steps of:
a) fermenting a medium containing a source of xylose with a cell as defined in any one of claims 5-13, and optionally,
b) recovery of the fermentation product.
